# EUROPEAN PATENT APPLICATION

(11) **EP 1 992 700 A1**
(43) Date of publication of application: **19.11.2008**
(21) Application number: 08009144.0
(22) Date of filing: 16.05.2008
(51) Int. Cl.: C12Q 1/44

(54) **Method for producing dry analytical element for pancreatic lipase measurement**

(30) Priority: 16.05.2007 JP 2007130502
(71) Applicant: FUJIFILM Corporation, Minato-ku Tokyo 106-0031 (JP)
(72) Inventor: Kageyama, Shigeki c/o Fuijfilm Corporation, Saitama 351-8585 (JP); Tanaka, Hideaki c/o Fuijfilm Corporation, Saitama 351-8585 (JP); Nakamura, Kentaro c/o Fuijfilm Corporation, Saitama 351-8585 (JP)
(74) Representative: Albrecht, Thomas

(57) **Abstract**

It is an object of the present invention to provide a method for producing a dry analytical element for pancreatic lipase analysis in a stable manner with good accuracy, such element having high specificity with respect to pancreatic lipase and being available for convenient use. The present invention provides a method for producing a dry analytical element for measurement of pancreatic lipase contained in a body fluid, said element comprising at least one development layer or reagent layer containing diglyceride or triglyceride of long-chain alkyl fatty acid having 14 to 20 carbon atoms, monoglyceride lipase, and a glycerine measurement reagent, which comprises a step of adding glyceride dissolved in lower alcohol or acetone to the development layer or reagent layer and drying the glyceride.

## Description

### Technical Field

The present invention relates to a method for producing a dry analytical element for measurement of lipase activity (particularly pancreatic lipase activity) in a liquid sample such as, in particular, serum and plasma of humans or animals, such analytical element being available for convenient use and having good accuracy. The dry analytical element produced in the present invention is particularly useful for diagnosing human and canine pancreatic diseases.

### Background Art

Pancreatic lipase analysis, which is useful for pancreatic disease diagnosis, is often carried out by measuring pancreatic lipase under conditions in which a micellar substrate is dispersed in water in view of the fact that pancreatic lipase functions in oil-water interface. This is because non-pancreatic lipase such as lipoprotein lipase, liver lipase, or esterase reacts with a substrate solubilized with a surfactant or the like or with glyceride of fatty acid having short alkyl chains. Thus, it is considered that a technically important point for development of dry analytical elements for pancreatic lipase is the incorporation of glyceride of a long-fatty-acid (serving as a substrate), which is specific to pancreatic lipase, into such analytical element, in a state that the glyceride is specific to pancreatic lipase.

Dry analytical elements used for lipase analysis are roughly classified into two groups. An example of a first group is a multilayer dry analytical element (JP Patent Publication (Kokai) No. 59-48098 A (1984)) according to a method wherein triglyceride is used as a substrate and is converted into a dye via glycerine and hydrogen peroxide. According to the first disclosed method, a triglyceride having a long-chain alkyl group having at least 8 carbon atoms at an ester position (α position) and having a short-chain alkyl group at two other esters each is used as a substrate, 1,2 diacetylglyceride generated by lipase in a specimen is converted into glycerine with the use of an esterase (namely, acetinase), and glycerine is converted into a dye. The above method is a convenient and highly accurate lipase measurement method. However, it has been reported that selectivity with respect to pancreatic lipase is not high, and thus the method is problematic for diagnosis of pancreatic diseases.

Next, a method using a dry chemistry reagent for pancreatic lipase analysis, such reagent comprising triglyceride (serving as a substrate) comprising a long chain fatty acid (e.g., triolein) having 14 to 20 carbon atoms, monoglyceride lipase, and a glycerine measurement reagent is disclosed in JP Patent Publication (Kokai) No. 4-316500 A (1992). Further, a method wherein a highly accurate multilayer analytical element is prepared according to the above method and fine particles are further incorporated into the element in order to improve lipase reactivity is disclosed in JP Patent Publication (Kokai) No. 2002-125699 A. In the method of JP Patent Publication (Kokai) No. 4-316500 A (1992), highly fat-soluble substrate is incorporated, so that a protective colloid such as gum Arabic is used for aqueous system dispersion using ultrasonic dispersion (JP Patent Publication (Kokai) No. 4-316500 A (1992): Examples). Accordingly, it is necessary to maintain the reproducibility of substrate dispersion and uniformity in particle size distribution. Also, it is thought that production of such element is difficult since no support is used. Further, in JP Patent Publication (Kokai) No. 2002-125699 A, a method for adding substrate is not mentioned.

As a method for adding substrate, for instance, JP Patent Publication (Kokai) No. 4-316500 A (1992) contains the following description: "triglyceride, such as triolein, comprising a long chain fatty acid in each of three ester positions has the property of being emulsified with difficulty. Thus, even if a solution in which triolein has been uniformly emulsified and dispersed via agitation or by physical shearing force generated by ultrasound waves or the like is added in the presence of a surfactant or a protective colloid upon preparation of a dry reagent, water serving as a dispersion medium disappears when the reagent becomes dry, and thus an emulsified product aggregates or coalesces so as to adhere to the surface of a development layer, resulting in significant reduction in the surface area in oil-water interface. Upon measurement, even if a specimen (liquid) containing lipase is allowed to react with such dry reagent, triolein remains in a state of aggregating or coalescing and thus does not return to the original state of being dispersed because of lack of physical shearing force. The reaction field of lipase is an oil-water interface. Thus, a decrease in the surface area of an oil-water interface is thought to cause a decrease in reaction rate."

An example of a second group is a dry analytical element obtained by a method using 1,2-O-dilauryl-rac-glycero-3-glutaric acid/resorufin ester serving as a dye-releasing substrate (JP Patent Publication (Kokai) No. 9-154598 A (1997)). Such method is a preferable because high specificity with respect to pancreatic lipase can be achieved and a glycerine coloring system is unnecessary. However, the substrate incorporated into a dry analytical element is highly likely to degrade. Thus, such dry analytical element has still not been available in practice, although it has been attempted to separate a low-pH layer containing a lipase substrate from another high-pH reagent layer. In addition, in such case, an ether system solvent that is thought to be preferable for dissolution of a substrate imposes significant environmental burdens and thus is seriously problematic in terms of production suitability in the present situation in which environmentally-friendly designs are strongly required. Further, the relatively high price of such a substrate is also problematic in terms of practical use.

As described above, the product disclosed in JP Patent Publication (Kokai) No. 59-48098 A (1984) is still the only commercially available dry analytical element for lipase analysis, although the product has low pancreatic lipase specificity. Thus, dry analytical elements that are excellent in terms of reliability for diagnosis of pancreatic diseases have been desired in the market.

### Disclosure of the Invention

It is an object of the present invention to provide a method for producing a dry analytical element for pancreatic lipase analysis in a stable manner with good accuracy, such element having high specificity with respect to pancreatic lipase and being available for convenient use, and to provide a dry analytical element for pancreatic lipase analysis prepared by the method. In order to achieve the above object, it is technically required to provide a method for incorporating long-chain-fatty-acid diglyceride or triglyceride (having substrate responsiveness to pancreatic lipase), such as triolein, which is finely dispersed when used in a water system according to a dissolution method, into a dry analytical element. The expression "substrate responsiveness to pancreatic lipase" means high specificity with respect to pancreatic lipase and high levels of enzymatic activity sufficient for the improvement in analysis accuracy.

In view of the above circumstances, the present inventors conducted intensive studies. As a result, they have completed the present invention with the use of: a substrate highly specific to pancreatic lipase; the constitution of a dry analytical element that can realize convenient and highly accurate analysis; a coloring reaction system that can plausibly carry out coloring with high reproducibility; and a method wherein a substrate (e.g., triolein) is added and incorporated via dissolution with the use of an organic solvent, such method having so far been unused.

The present invention provides a method for producing a dry analytical element for measurement of pancreatic lipase contained in a body fluid, said element comprising at least one development layer or reagent layer containing diglyceride or triglyceride of long-chain alkyl fatty acid having 14 to 20 carbon atoms, monoglyceride lipase, and a glycerine measurement reagent, which comprises a step of adding glyceride dissolved in lower alcohol or acetone to the development layer or reagent layer and drying the glyceride.

Preferably, the drying of glyceride is hot-air drying.

Preferably, glyceride is dissolved in methanol, ethanol, propyl alcohol, or acetone. Preferably, glyceride is triglyceride.

Preferably, triglyceride is triolein.

Preferably, monoglyceride lipase is *Bacillus stearothermophilus* H-165-derived monoglyceride lipase.

Preferably, the glycerine measurement reagent comprises glycerol kinase, glycerophosphate oxidase, and a coloring reagent.

Preferably, the dry analytical element is composed of a water-impermeable support, a reagent layer, and a development layer.

Preferably, the development layer is made of fabric or a porous membrane.

Preferably, the porous membrane is a porous membrane formed with polyvinylsulfone or acetylcellulose or a porous membrane formed with microbeads.

Preferably, the development layer is made of fabric.

Preferably, the amount of monoglyceride lipase added is 8000 U/m² to 1000 U/m².

Another aspect of the present invention provides a dry analytical element for measurement of pancreatic lipase contained in a body fluid, which is produced by the method according to the present invention as mentioned above.

Further another aspect of the present invention provides a method for measuring pancreatic lipase in a body fluid, which comprises applying the body fluid to the dry analytical element for measurement of pancreatic lipase contained in a body fluid which is produced by the method according to the present invention as mentioned above. Preferably, the body fluid is canine blood.

According to the present invention, it is possible to produce a dry analytical element for pancreatic lipase analysis in a stable manner with good accuracy, such element being highly specific to pancreatic lipase and available for convenient use.

### Brief Description of the Drawings

Fig. 1 shows a reaction time course for a pancreatic lipase solution on the slide of Example 1.
Fig. 2 shows a reaction time course for a 500 U/L human pancreatic lipase solution (o: dissolved in a 7%human albumin solution), and that for a 7% human albumin solution (•), when each solution (10 µl) was separately spotted on the lipase analytical element prepared in Example 2 with the use of triolein or linolyl diglyceride and heated at 37°C, followed by measurement at 650 nm.
Fig. 3 shows the results of measurement of reflection optical density at 650 nm while a dry analytical element containing a glycerine coloring system was being heated at 37°C, such dry analytical element being prepared by adding an ethanol solution thereto, allowing the analytical element to stand at room temperature for a certain period of time denoted in the figure (0 to 20 minutes), carrying out vacuum drying (fig. left) and hot-air drying (fig. right), and then spotting 3 mM glycerine (10 µl) thereon.
Fig 4 shows the results of examination of influences of reduction in the amount of monoglyceride lipase (MGLP) on a chylous specimen.
Fig.5 shows the result when tristearin was used as a substrate.
Fig 6 shows the correlation of many specimens in the dry analytical element of the present invention when using canine serum. In the control method, a solution method (Roche) using 1,2-O-dilauryl-rac-glycero-3-glutaric acid/resorufin ester as a substrate was used.

### Best Mode for Carrying Out the Invention

The method of the present invention is a method for producing a dry analytical element for measurement of pancreatic lipase contained in a body fluid, such element comprising at least one development layer or reagent layer containing diglyceride or triglyceride of a long-chain alkyl fatty acid having 14 to 20 carbon atoms, monoglyceride lipase, and a glycerine measurement reagent, such method comprising a step of adding glyceride dissolved in lower alcohol or acetone to the development layer or reagent layer and drying the glyceride.

According to the present invention, a substrate is diglyceride or triglyceride (and preferably triglyceride) comprising a long chain fatty acid having a high specificity with respect to pancreatic lipase. More preferably, triolein is used. In addition, monoglyceride lipase that does not substantially react with diglyceride, and a conventional glycerine coloring reagent that is highly reliable are used. A layer structure may comprise at least one development layer or reagent layer. A test paper formed in a single layer may also be used. Preferably, in order to improve measurement accuracy and strength, a multilayer analytical element comprising a support and at least one reagent layer is used. A substrate is obtained by dissolving glyceride such as triolein in an organic solvent such as ethanol, and the resultant is incorporated into a dry analytical element, preferably followed by hot-air drying. As a result, it becomes possible to produce a dry analytical element where an enzyme involved in a glycerine coloring reaction is not damaged even with the addition of an organic solvent; a specificity with pancreatic lipase is high and a reactivity with pancreatic lipase is sufficiently high; and a glyceride oil-water interface can be formed.

Further, in the cases of some specimens that had been preserved for a relatively long period of time (which were so-called chylous specimens containing lipoprotein at a high concentration), abnormal data that was thought to result from lipoprotein degradation was observed. However, such error was significantly reduced by controlling the amount of monoglyceride lipase added as a conjugated enzyme in the present invention.

A light-permeable and/or water-impermeable support can be used to constitute a support layer of the dry analytical element for lipase measurement of the present invention. Examples of a light-permeable/water-impermeable support include a film- or sheet-type transparent support having a thickness of approximately 50 µm to 1 mm and preferably approximately 80 µm to 300 µm and comprising polymers such as polyethylene terephthalate, polycarbonate of bisphenol A, polystyrene, cellulose ester (e.g., cellulose diacetate, cellulose triacetate, or cellulose acetate propionate), or the like. The support is useful in view of rapid and stable production of the dry analytical element for lipase measurement.

An undercoat layer is provided on the surface of a support according to need such that adhesion between a reagent layer provided on the support and the support can be strengthened. In addition, physical or chemical activation treatment is carried out on the support surface, instead of provision of an undercoat layer, such that adhesivity can be improved.

A reagent layer is provided on a support (via another layer such as an undercoat layer in some cases). A reagent layer is a water-absorbing and water-permeable layer containing a hydrophilic polymer binder in which at least a portion of a reagent composition described below, such composition reacting with lipase serving as an analyte so as to cause optically detectable changes, is substantially uniformly dispersed. This layer has a function of converting glycerin into dye, and is therefore referred to as a reaction layer or a coloring layer.

A hydrophilic polymer that can be used as a binder for a reagent layer is generally a natural or synthetic hydrophilic polymer having a swelling rate at 30°C in the range of approximately 150% to 2000% and preferably of approximately 250% to 1500%. Examples of such hydrophilic polymer include gelatins (e.g., acid-treated gelatin and deionized gelatin), gelatin derivatives (e.g., phthalated gelatin and hydroxyacrylate graft gelatin), agarose, pullulan, pullulan derivatives, polyacrylamide, polyvinyl alcohol, and polyvinylpyrrolidone, which are disclosed in JP Patent Publication (Kokai) No. 58-171864 A (1983), JP Patent Publication (Kokai) No. 60-108753 A (1985), and the like.

A reagent layer may be a layer that has been appropriately cross-linked and insolublized using a crosslinking reagent. Examples of a crosslinking reagent include: conventional vinyl sulfone crosslinking reagents (such as 1,2-bis(vinyl sulfonylacetamide) ethane and bis(vinyl sulfonylmethyl) ether) and aldehyde and the like, for gelatin; and aldehyde and epoxy compounds comprising two glycidyl groups and the like, for methallyl alcohol copolymer.

The thickness of a reagent layer when dried is preferably in the range of approximately 1 µm to 100 µm and more preferably of approximately 3 µm to 30 µm. Preferably, a reagent layer is substantially transparent.

The development layer has a function of uniformly diffusing spotted body fluid, and also may have a function of performing a reaction of lipase in body fluid and a substrate in a development layer and a subsequent reaction of intermediate products. Therefore, the development layer may be referred to as development reaction layer.

According to the present invention, it is preferable to use a fabric development layer as a development layer. Alternatively, it is also possible to use a non-fabric material such as a porous membrane comprising polyvinylsulfone or acetylcellulose, porous membrane formed with microbeads, glass fiber filter paper, or filter paper.

Examples of such porous development layer made of fabric include woven fabric development layers (e.g., plain weave fabric such as broadcloth or poplin) described in JP Patent Publication (Kokai) No. 55-164356 A (1980), JP Patent Publication (Kokai) No. 57-66359 A (1982), and the like; knitted fabric development layers (e.g., tricot knitted fabric, double tricot knitted fabric, and Milanese knitted fabric) described in JP Patent Publication (Kokai) No. 60-222769 A (1985) and the like; and a development layer comprising woven fabric or knitted fabric subjected to etching treatment with an alkaline etching solution described in JP Patent Publication (Kokai) No. 1-172753 A (1989). Knitted fabric is preferable. In particular, tricot knitted fabric is preferable. Examples of fabric material used include polyester, cotton, nylon, silk, vinylon, rayon, polyamide, acrylic, wool, polypropylene, and hemp. Preferably, polyester is used. The appropriate thickness of a development layer is approximately 50 to 400 µm and preferably approximately 200 to 300 µm. The porosity of fabric is approximately 20% to 90% and preferably approximately 40% to 85%.

In the cases of woven fabric and knitted fabric used for a porous development layer, it is possible to improve the adhesivity of such fabric to a lower layer (close to a support) by carrying out a physical activation treatment represented by a glow discharge treatment or corona discharge treatment disclosed in JP Patent Publication (Kokai) No. 57-66359 A (1982) on at least one side of the fabric or by hydrophilizing the fabric in a manner such that a washing and degreasing treatment and/or a hydrophilization treatment involving surfactant dipping, hydrophilic polymer dipping, or the like, which are disclosed in JP Patent Publication (Kokai) No. 55-164356 A (1980), JP Patent Publication (Kokai) No. 57-66359 A (1982) and the like, are carried out, or such that a treatment involving an appropriate combination of the above treatments is carried out in a sequential manner.

When a porous layer is used as a development reaction layer, a porous medium thereof may be fibrous or nonfibrous. Examples of a fibrous material that can be used include filter paper, nonwoven fabric, woven fabric (e.g., plain weave fabric), knitted fabric (e.g., tricot knitted fabric), and glass fiber filter paper. Examples of a nonfibrous material include a membrane filter comprising cellulose acetate and the like disclosed in JP Patent Publication (Kokai) No. 49-53888 A (1974), and a particulate unit layer having continuous voids, such layer comprising fine particles of an inorganic or organic substance disclosed in JP Patent Publication (Kokai) No. 49-53888 A (1974), JP Patent Publication (Kokai) No. 55-90859 A (1980) (corresponding to US Patent No. 4,258,001), JP Patent Publication (Kokai) No. 58-70163 A (1983) (corresponding to US Patent No. 4,486,537), and the like. Also, layer laminated products having a plurality of porous layers that partially adhere to each other disclosed in the following documents and the like are preferable: JP Patent Publication (Kokai) No. 61-4959 A (1986) (corresponding to EP 0166365 A); JP Patent Publication (Kokai) No. 62-116258 A (1987); JP Patent Publication (Kokai) No. 62-138756 A (1987) (corresponding to EP 0226465 A); JP Patent Publication (Kokai) No. 62-138757 A (1987) (corresponding to EP 0226465 A); and JP Patent Publication (Kokai) No. 62-138758 A (1987) (corresponding to EP 0226465 A).

In order to add reagents to a development layer, a development layer is first formed and then reaction reagents may be added by means of coating or the like. Alternatively, an example of a useful method is the method described in JP Patent Publication (Kokai) No. 55-164356 A (1980), comprising of dipping or coating to a porous membrane composed of paper, fabric or the like with the reagents of the present invention and allowing the resultant to adhere to another water-permeable layer formed on a support.

A porous layer may be a development layer having a so-called measuring function that allows a supplied liquid to be developed in an area that is almost in proportion to the amount of the liquid. It is effective to control such function with the use of surfactants and hydrophilic binders.

It is also possible to provide a layer that differs from the above layers to the dry analytical element of the present invention. Examples thereof include a light-shielding layer, a water-absorbing layer, and an adhesive layer.

In the measurement reaction system used in the present invention, diglyceride or triglyceride serving as a substrate is degraded with lipase to be measured, and monoglyceride generated upon such degradation is degraded with monoglyceride lipase. L-α-glycerophosphate is generated from the glycerol with the use of glycerol kinase. Then, L-α-glycerophosphate is turned into dihydroxyacetone phosphate with L-α-glycerophosphate oxidase, and also hydrogen peroxide is generated. Coloring from a coloring dye is induced by the function of peroxidase with the use of hydrogen peroxide.

A substrate to be used is diglyceride or triglyceride. Preferably, such substrate is triglyceride, which serves as a natural pancreatic lipase substrate. This is because diglyceride is likely to become less specific to pancreatic lipase since the activity of non-pancreatic lipase such as lipoprotein lipase or esterase in blood is not negligible. In addition, diglyceride, even in a small amount, reacts with monoglyceride lipase, and thus the background level upon analysis tends to become high, which is problematic.

Triglyceride has three ester positions, each having long chain fatty acid via an ester bond. A triglyceride which is highly selective to pancreatic lipase is preferable. Each long chain fatty acid has approximately 14 to 20 and preferably 16 to 20 carbon atoms. Examples thereof include saturated fatty acids such as palmitic acid and stearic acid, and unsaturated fatty acids such as oleic acid, linoleic acid, and linolenic acid. Specific examples of triglyceride include triolein, trimyristin, tripalmitin, tristearin, trilinolein, 1-myristodipalmitin, 1-palmitodistearin, 2-palmitodistearin, and 1-stearo-2-pahnito-3-myristin. Triglyceride of unsaturated fatty acids is preferred. Triolein is particularly preferable. Tristearin which is composed of unsaturated fatty acids is not good in view of lipase reactivity in the produced dry analytical element for lipase measurement, as compared with triolein.

Monoglyceride lipase is added to a reagent system that is incorporated into the dry analytical element of the present invention. A preferred example of monoglyceride lipase is one that does not substantially react with triglyceride and diglyceride but reacts with monoglyceride of long chain fatty acid. *Bacillus stearothermophilus* H-165-derived monoglyceride lipase described in JP Patent Publication (Kokai) No. 63-245672 A (1988) and JP Patent Publication (Kokai) No. 4-316500 A (1992) is particularly preferable.

Glycerol kinase allows glycerol and ATP to react with each other so as to change them into L-α-glycerophosphate (L-glycerol-3-phosphate) and ADP, respectively. It uses Mg²⁺ and Mn²⁺ as coenzymes.

L-α-glycerophosphate oxidase (glycerol-3-phosphate oxidase) oxidizes L-glycerophosphate so as to change it into dihydroxyacetone phosphate and generate hydrogen peroxide.

Various coloring systems in which coloring is caused by the function of peroxidase with the use of hydrogen peroxide have been developed for dry analytical elements. Thus, it is possible to appropriately select and use one thereof. Most of them are leuco dyes, and typical examples thereof include o-toluidine. Diallylimidazole leuco dyes are preferred, but Trinder reagent may be used.

In order to increase reactivity of pancreatic lipase (mainly contained in blood) that serves as a substance to be measured of the present invention, it is preferable to add colipase to a reagent system incorporated into the dry analytical element of the present invention. A preferred example of colipase is pig-pancreas-derived colipase. In addition, in order to increase the activity of pancreatic lipase and to reduce the lipase activity of non-pancreatic lipase, deoxycholic acid or taurocholic acid may be added as an activating reagent. Thus, influences of esterase, liver lipase, and lipoprotein lipase are removed, and thus pancreatic lipase can be measured with high specificity.

The contents of the above reagents are as follows:
triglyceride: approximately 0.1 to 15 g/m² and preferably approximately 0.5 to 10 g/m²;
glycerol kinase: 0.5 to 100 KU/m² and preferably approximately 1 to 10 KU/m²;
L-α-glycerophosphate oxidase: approximately 2 to 200 KU/m² and preferably approximately 5 to 50 KU/m²;
peroxidase: approximately 1 to 200 KU/m² and preferably approximately 5 to 50 KU/m²;
monoglyceride lipase: approximately 2 to 100 KU/m² and preferably approximately 3 to 30 KU/m²;
coloring dye: approximately 0.05 to 2.00 g/m² and preferably approximately 0.1 to 1.00 g/m²;
colipase: preferably 0.010 to 0.400 g/m² (5 to 200 KU/m²); and
deoxycholic acid: approximately 0.1 to 10 g/m².

Monoglyceride lipase used herein is in an amount of preferably 8000 U/m² to 1000 U/m², more preferably 6000 U/m² to 2000 U/m², and the most preferably 4300 U/m² to 2000 U/m². Although monoglyceride lipase is a conjugated enzyme, it is not preferable to add it in an excessive amount. When diglyceride is used as a substrate, the background level might be increased. In addition, even when triglyceride is used as a substrate, the reaction of a part of lipoprotein in blood is induced along with increases in the amount of monoglyceride lipase, resulting in the generation of measurement errors.

The total amount of such reagent composition may be contained in a reagent layer or development layer. Alternatively, it may be divided such that it is contained in both layers, or it may be partially contained in another layer.

It is also possible to add other reagents, such as a buffer and a surfactant, to the dry analytical element of the present invention.

Examples of a buffer that can be contained in the dry analytical element of the present invention include known buffers such as a carbonate buffer, a borate buffer, a phosphate buffer, a tris salt buffer, and a Good's buffer. These buffers can be selected and used by referring to known references such as "Primary Experimental Methods for Proteins and Enzymes (Tanpakushitsu/Koso no Kiso Jikken-hou)" (Takeichi Kajio et al., Nankodo Co., Ltd., 1981). The content thereof may be approximately equal to that generally used in an integrated multilayer analytical element, which is in the range of approximately 100 mg/m² to 5.0 g/m² and preferably of approximately 500 mg/m² to 3.0 g/m².

A development layer or reaction layer of the analytical element of the present invention may contain a surfactant such as a nonionic surfactant. A surfactant used contains a combination of a lipophilic group (e.g., an alkyl group, an alkylphenyl group, a styrenated phenyl group, a benzilphenyl group, or a sorbitanalkyl group), and a hydrophilic group (a polyoxyethylene group, or a polyglycerol group, and a polyoxyethylenepolypropylene polymer). Examples of such surfactant include polyoxyethylene alkylether, polyoxyethylene branched alkylether, polyoxyalkylene alkylether, polyoxyethylene alkylphenylether, and alkylphenyl polyglyceride. Specific examples thereof include polyoxyethylene tridecylether, polyoxyethylene branched decylether, polyoxyethylene p-octylphenyl ether, polyoxyethylene p-octylphenyl ether, polyoxyethylene p-nonylphenyl ether, polyoxyethylene oleyl ether, polyoxyethylene sorbitan monolaurate, p-nonylphenoxypolyglycidol, and octylglucoside. Among such nonionic surfactants, polyoxyethylene tridecylether, polyoxyethylene branched decylether, p-octylphenoxypolyethoxyethanol, p-nonylphenoxypolyethoxyethanol, p-nonylphenoxypolyglycidol, and the like are preferable. A nonionic surfactant is allowed to be contained in a development layer such that a function of developing an aqueous liquid sample (metering function) is further improved. A nonionic surfactant is allowed to be contained in a reaction layer, thereby water contained in an aqueous liquid sample is facilitated to be absorbed to a reaction layer in a substantially uniform manner upon analysis operations. Also in such case, the liquid comes into contact with a development layer in a rapid and substantially uniform manner.

In addition, a development layer may also contain a hydrophilic polymer. Examples of a hydrophilic polymer include starch, cellulose, cellulose derivatives (e.g., methylated cellulose, hydroxyethylated cellulose, and hydroxypropylated cellulose), agarose, gelatins (e.g., an acid-treated gelatin and a deionized gelatin), gelatin derivatives (e.g., a phthalated gelatin and a hydroxyacrylate graft gelatin), acrylamide polymers, copolymers each comprising acrylamide and a various vinyl monomer, vinylpyrrolidone polymers, copolymers each comprising vinylpyrrolidone and a various vinyl monomer, acrylate polymers, and copolymers each comprising acrylate and a various vinyl monomer. Among the above hydrophilic polymers, vinylpyrrolidone derivatives and cellulose derivatives are preferable.

A preferred production method is described below. A binder such as gelatin and a surfactant are added to a support. A water-soluble coating solution having improved film-forming properties is coated thereto and dried, so that a reagent layer is prepared. A development layer is obtained as follows when fabric or a formed porous membrane is used as a development layer. Water is added to a part of a reagent layer such that the part is solubilized. A binder is further softened (by heating according to need) and then it is fixed by applying pressure to a development layer membrane and a reagent layer on the support, followed by drying.

Hitherto, in the case of a method for dispersing triolein in water, which is used as a method for adding triolein to an analysis solution or dry analysis apparatus for lipase, the glyceride particle size is unstable due to changes in terms of the time for the addition of a reagent, the stirring efficiency, the solution temperature, and the like, although the particles are stabilized with a surfactant or a protective colloid. Thus, intra-lot or lot-to-lot variations are significant so that it is difficult to produce a dry analysis apparatus for lipase with good accuracy. Further, it is believed that an expensive and advanced dispersion apparatus is necessary for such dispersion and emulsification. In addition, particle sedimentation occurs unless the size of dispersed particles becomes small. Such sedimentation is problematic for preparation of uniform analysis apparatuses.

According to a method for solubilizing triglyceride or diglyceride in a water-soluble solution containing a surfactant so as to add the solution to a dry analysis apparatus, not only the reactivity of pancreatic lipase but also the reactivity of non-pancreatic lipase or esterase to the substrate thereof may be increased in some cases. In addition, a surfactant might deactivate a conjugated enzyme. Such outcomes are not preferable.

Thus, if necessary in order to dissolve glyceride (lipase substrate) such as triolein in a lower alcohol or acetone so as to improve coating properties, a binder such as polyvinylpyrrolidone is added to control viscosity. Preferably, such lower alcohol or acetone is a lower alcohol solvent having 1 to 6 carbon atoms or acetone. In particular, methanol, ethanol, propyl alcohol, and acetone are preferable. Among them, ethanol is particularly preferable. It is not preferable to use an ether solvent such as tetrahydrofuran or dioxane. One reason for this is that a water soluble polymer (e.g., polyvinylpyrrolidone) that is useful for improved coating properties and improved developing property of body fluid becomes less soluble in a case involving the use of such an ether solvent. Chloroform and methylene chloride are useful because glyceride dissolves well therein. However, in recent years, environmental toxicity such as carcinogenicity has been problematic, so that the use thereof requires attention and is not preferred.

Regarding the ratio of the amount of glyceride to that of the solvent, preferably, 1 g of triolein is used for 50 to 300 g of ethanol when triolein is dissolved in ethanol, for example. More preferably, 1 g of triolein is used for 100 to 200 g of ethanol. When the triolein concentration is high, triolein is not dissolved in ethanol, and thus it becomes difficult to uniformly add triolein. For a method for adding glyceride to a solvent solution, coating or dipping may be carried out. Regarding such method for adding glyceride, an efficient and uniform production method is a method comprising a step of using a coater device for coating and drying. In such step, drying is preferably hot-air drying. Drying air is at a temperature of preferably 20°C to 60°C and particularly preferably 25°C to 40°C. Preferably, a dew point is 0°C to 10°C. Preferably, an air flow is 0.5 to 10 m/second. A required time period for drying is a time period during which a solvent is substantially dried. Meanwhile, drying for a long period of time may result in denaturing of a conjugated enzyme, and thus the time period for drying is preferably 1 to 60 minutes. It is also possible to set preferable drying conditions by controlling the temperature, dew point, air speed, and direction of drying air and a time period for drying in each of a plurality of drying zones.

Calcium chloride (CaCl₂) may be added to any coating solution. However, it may react with deoxycholic acid and form an aggregate in some cases. As CaCl₂ can be dissolved in ethanol, it is preferred that CaCl₂ is dissolved in a substrate ethanol solution and is then added.

A reaction reagent that is not dissolved in an organic solvent is separately added by applying it as a water-soluble solution. In order to improve coating suitability and blood development properties, it is preferable to add a binder and a surfactant. A pH buffer, colipase serving as a lipase reaction promoter, and deoxycholic acid may be added to such solution. As above, a reagent is added to a development layer via coating and drying.

Basically, each reaction reagent may be added to any layer upon production, provided that reagent conditions appropriate for reaction can be achieved upon reaction of lipase in a specimen via dissolution and dispersion.

Regarding a method for adding a reagent, dipping or spraying may be carried out as long as a uniform amount of a reagent can be determined. Regarding the order of preparation of individual layers, a method whereby a uniform layer in which a reagent is not degraded is obtained may be used. In some cases, it is also possible to prepare a layer according to the above production method with the use of a porous membrane such as glass fiber filter paper or filter paper and without the use of a support. Also in such case, it is preferable to dry a solution obtained by dissolving a substrate in an organic solvent via hot-air drying.

In view of production, packaging, transportation, storage, measurement operations, and other points, it is preferable to use the integrated multilayer analytical element of the present invention in a manner such that it is cut into square pieces having sides each approximately 10 mm to 30 mm in length or circular pieces having sizes similar to the sizes of the square pieces, following which the pieces are accommodated in slide frames or the like disclosed in the following documents so as to be used as analytical slides: JP Patent Publication (Kokai) No. 57-63452 A (1982); JP Patent Publication (Kokai) No. 54-156079 A (1979); JP Utility Model Publication (Kokai) No. 56-142454 U (1981); JP Utility Model Publication (Kokai) No. 58-32350 U (1983); and JP Patent Publication (Kokai) No. 58-501144 A (1983).

The integrated multilayer analytical element of the present invention is used as follows. An aqueous liquid sample in an amount of approximately 5 µl to 30 µl, and preferably approximately 8 µl to 15 µl, is supplied by spotting to a porous development layer according to the methods according to the above documents. If necessary, incubation is carried out at a substantially constant temperature in the range of approximately 20°C to 45°C. Then, reflective photometry is carried out from the light-permeable support side of the integrated multilayer analytical element in order to observe detectable changes therein, including color change and coloring. Thereafter, the components to be measured in a liquid sample are analyzed based on the principles of colorimetric methods.

The present invention is hereafter described in greater detail with reference to the following examples, although the technical scope of the present invention is not limited thereto.

### Examples

### Example 1: Preparation of a dry analytical element with the use of triolein as a substrate:

### (1) Addition of a glycerine coloring reagent:

A reagent of the following composition was coated as an aqueous solution to a gelatin-undercoated polyethylene terephthalate film having a thickness of 180 µm which was smooth, colorless, and transparent, followed by drying. Subsequently, water was uniformly supplied to the film such that the film became wet. Tricot knitted fabric prepared by knitting (36 gauge) with polyethylene terephthalate spun yarn (corresponding to 50 deniers) was laminated thereon by light pressurization. The gelatin was solidified at a drying temperature of 20°C, followed by drying at 45°C. In addition, the coating solution comprising a pH buffer (PIPES) used was adjusted to have a pH of 6 with a 1 N-NaOH aqueous solution.
Gelatin: 12 g/m²
PIPES (Dojindo Laboratories): 22 g/m²
Magnesium chloride (Wako Pure Chemical Industries, Ltd.): 0.52 g/m²
ATP-2 sodium salt (Oriental Yeast Co., Ltd.): 1.4 g/m²
Polyoxyethylene tridecylether HLB14.8 (Dai-ichi Kogyo Seiyaku Co., Ltd.): 0.55 g/m²
Polyethylene alkyl branched decylether HLB15.9 (Dai-ichi Kogyo Seiyaku Co., Ltd.): 0.059 g/m²
Leuco dye: 0.21 g/m²
Horseradish peroxidase (TOYOBO Co., Ltd): 14 KU/m²
Glycerol kinase (Asahi Kasei Corporation): 3.8 KU/m²
L-α-glycerophosphate oxidase (Asahi Kasei Corporation): 19 KU/m²
1,2-bis(vinylsulfonylacetamide) ethane: 3.3 g/m²

### (2) Addition of a substrate:

A reagent of the following composition was dissolved in ethanol and coated to the above fabric, followed by hot-air drying at a drying temperature of 32°C for 15 minutes with the use of air at a dew point of 0°C. The air speed was 0.5 m/minute (1 minute) and 5 m/minute (15 minutes). In this Example, triolein (1.1 g) was dissolved with the addition of ethanol (175 g).
Calcium chloride (Wako Pure Chemical Industries, Ltd.): 0.18 g/m²
Polyvinylpyrrolidone K90 (BASF): 2.0 g/m²
Triolein (98%, ICN-Biochemical): 1.1 g/m²

### (3) Addition of a lipase reaction adjuvant

Further, the following reagent was dissolved in water and coated to the above resultant, followed by drying. Thus, a dry analytical element for pancreatic lipase was prepared. In addition, the coating solution comprising a pH buffer (HEPES) was adjusted to have a pH of 8.0 with the use of a 1 N-NaOH aqueous solution.
HEPES (Dojindo Laboratories): 6.1 g/m²
Sodium deoxycholate (Wako Pure Chemical Industries, Ltd.): 4.6 g/m²
Sodium taurodeoxycholate : 1.5 g/m²
Metolose: 2.1 g/m²
Monoglyceride lipase(Asahi Kasei Corporation): 4300 U/m²
Pig colipase (Roche) 0.051 g/m²
Ascorbate oxidase (TOYOBO Co., Ltd): 8500 U/m²

### (Measurement)

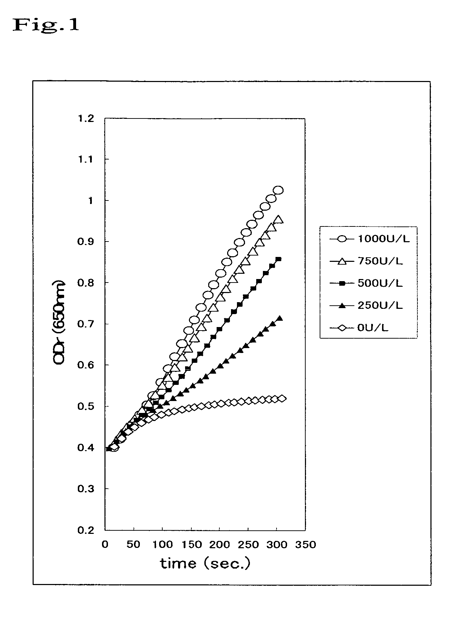

A solution (10 µl) obtained by adding a human lipase (Asahi Kasei Pharma Corporation) to serum was spotted onto the dry analytical element for pancreatic lipase prepared in Example 1, followed by heating at 37°C. Then, changes in coloring concentration were examined. The measurement apparatus used was a commercially available Fuji Dry Chem 5500. Fig. 1 shows the measurement results. It was confirmed that a coloring reaction takes place in a manner dependent on pancreatic lipase concentration.

### Example 2: Preparation of a dry analytical element with the use of diglyceride as a substrate:

A dry analytical element for pancreatic lipase was prepared in the manner described in Example 1, except that triolein added as a substrate in the experiment of Example 1 was replaced by 1,2-diglyceride as described below. In Example 2, ascorbate oxidase used in Example 1 was not added.

### (2) Addition of a substrate:

A reagent of the following composition was dissolved in ethanol and coated to the above fabric, followed by hot-air drying at a drying temperature of 32°C with air at a dew point of 0°C.
Calcium chloride (Wako Pure Chemical Industries, Ltd.): 0.18 g/m²
Polyvinylpyrrolidone K90: 2.0 g/m²
1,2-dilinoyl glycerol (Asahi Kasei Pharma Corporation): 1.1 g/m²

### (Measurement)

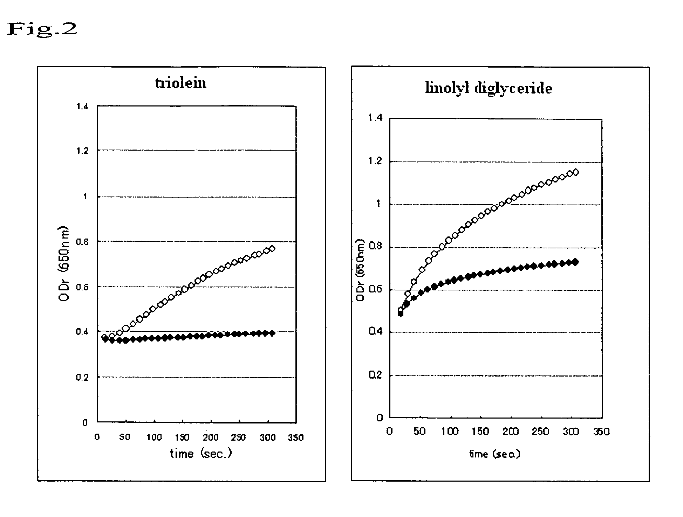

A solution obtained by adding a human lipase (Asahi Kasei Pharma Corporation) to serum (10 µl) was spotted onto the dry analytical element for pancreatic lipase prepared in Example 2, followed by heating at 37°C. Then, changes in coloring concentration were examined. The measurement apparatus used was a commercially available Fuji Dry Chem 5500. Fig. 2 shows the measurement results. As shown in fig. 2, coloring with pancreatic lipase was confirmed also in the case of linolyl diglyceride (which is diglyceride), as in the case of triolein. The results of a high background of reaction (coloring with a 7% human albumin solution) were obtained.

### Example 3: Comparison of solvents for substrates and drying conditions

In order to examine solvents for substrates and drying conditions, the following solvents were separately added to cloth laminate sheets obtained by adding the glycerine coloring reagent of Example 1 (the amount of addition was equal to that in Example 1), followed by immediate drying by the following drying method: a vacuum drying method or a hot-air drying method at room temperature (as in the case of Example 1). Alternatively, the sheets were allowed to stand at room temperature for 3 to 20 minutes before drying. For evaluation, a 7% human albumin solution containing 3 mM glycerine (10 µl) was spotted thereon, followed by heating at 37°C. Then, the reflection optical density at 650 nm was measured such that a reaction involving conversion of glycerine into a dye was measured. The measurement apparatus used was a commercially available Fuji Dry Chem 5500.

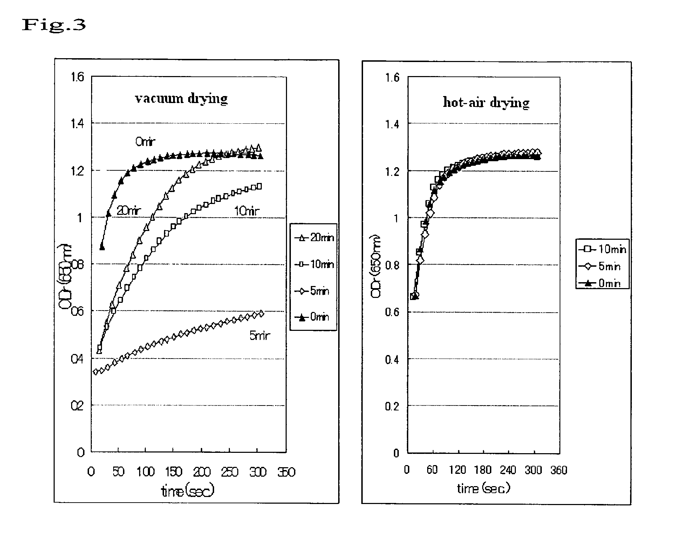

Fig. 3 shows an example indicating measurement results obtained by comparing results in the case of hot-air drying and those in the case of vacuum drying with the use of ethanol as a solvent. When ethanol was used, very poor glycerine coloring reaction was obtained, depending on the time period for standing still before drying in the case of vacuum drying. On the other hand, in the case of hot-air drying, no such influence was observed, even after drying after standing still for at least 10 minutes.

Table 1 shows the influence on a glycerine coloring reagent with the use of available solvents. It was possible to use ethanol, methanol, isopropyl alcohol, and acetone. In particular, ethanol and methanol were found to be preferable for hot-air drying.

**Table 1: Comparison of drying conditions and solvents**

| Solvent | Drying | Glycerine coloring | Background | Environmental |
|---|---|---|---|---|
| | | | (Reflection OD) | toxicity (#1) |
| Ethanol | Hot-air drying | Not influenced (good) | 0.4 | O |
| Ethanol | Vacuum drying | Influenced (poor) | 0.4 | O |
| Methanol | Hot-air drying | Not influenced (good) | 0.4 | Δ |
| Methanol | Vacuum drying | Influenced (poor) | 0.4 | Δ |
| Isopropyl alcohol | Vacuum drying | Not influenced (good) | 0.6 (occurrence of a nonspecific reaction) | Δ |
| Acetone | Hot-air drying | Not influenced (good) | 0.4 | Δ |
| Acetone | Vacuum drying | Not influenced (good) | 0.4 | Δ |

| | | | | |
|---|---|---|---|---|
| Environmental toxicity (#1): O : good Δ: problematic at the time of production | | | | |

### Example 4: Reduction of influence of a decrease in the amount of monoglyceride lipase (MGLP) on a chylous specimen

For analysis, dry analytical elements were prepared by changing the amount of monoglyceride lipase according to the table below without the addition of triolein used as a substrate in Example 1.

**Table 2:**

| Sample number | Amount of MGLP (U/m²) |
|---|---|
| 4-1 | 64 k |
| 4-2 | 32 k |
| 4-3 | 16 k |
| 4-4 | 8 k |

### (Measurement)

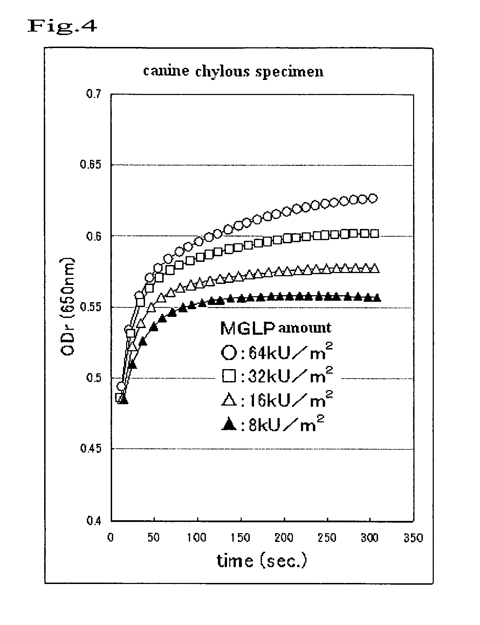

A canine chylous specimen (10 µl) containing substantially no lipase was spotted onto the pancreatic lipase dry analytical element prepared in Example 3, followed by heating at 37°C. Then, changes in coloring concentration were examined. The measurement apparatus used was a commercially available Fuji Dry Chem 5500. Fig. 4 shows the measurement results.

As a result, it was confirmed that when the amount of MGLP amount was obviously excessive, a non-lipase substance caused a coloring reaction in a canine chylous specimen. In addition, it was confirmed that a positive error was generated as a result of a mild OD increase during a time period from 180 to 300 seconds for lipase activity measurement. It was confirmed that the amount of MGLP added is preferably 8 kU/m².

### Example 5: Preparation of a dry analytical element with the use of tristearin as a substrate:

A dry analytical element for lipase measurement was prepared in the manner described in Example 1, except that tristearin was used in place of triolein used as a substrate in Example 1, and the coating amount of substrate liquid was set as mentioned below. Ethanol was used as a solvent for a substrate as in Example 1, and 174g of ethanol was added to 1.14g of tristearin. However, the solubility of the substrate was bad, and therefore it was coated as a suspension. Therefore, in order to suppress aggregation of the coating solution, the amount of polyvinylpyrrolidone was increased.
Calcium chloride (Wako Pure Chemical Industries, Ltd.): 0.18 g/m²
Polyvinylpyrrolidone K90 (BASF): 10.0 g/m²
Tristearin (Sigma): 1.1 g/m²

### (Measurement)

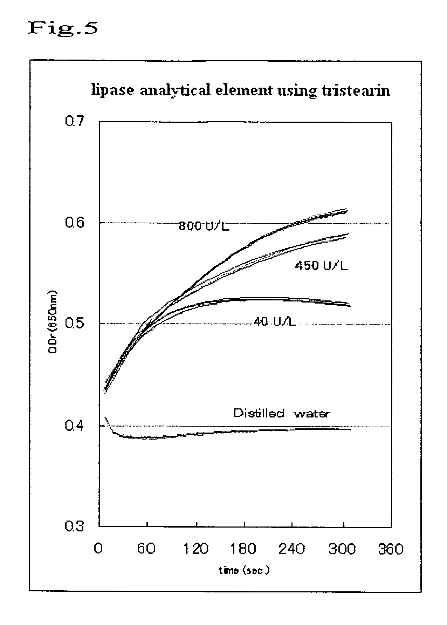

A sample (10 µl) obtained by adding human lipase (Asahi Kasei Pharma Corporation) to canine serum was spotted onto the pancreatic lipase dry analytical element prepared in Example 4, followed by heating at 37°C. Then, changes in coloring concentration were examined. The measurement apparatus used was a commercially available Fuji Dry Chem 7000. The results of the measurement are shown in Fig. 5. It was confirmed that a coloring reaction takes place in a manner dependent on pancreatic lipase concentration. The reactivity of lipase was lower than in the case of triolein.

### Example 6: Correlation of many specimens when using canine serum.

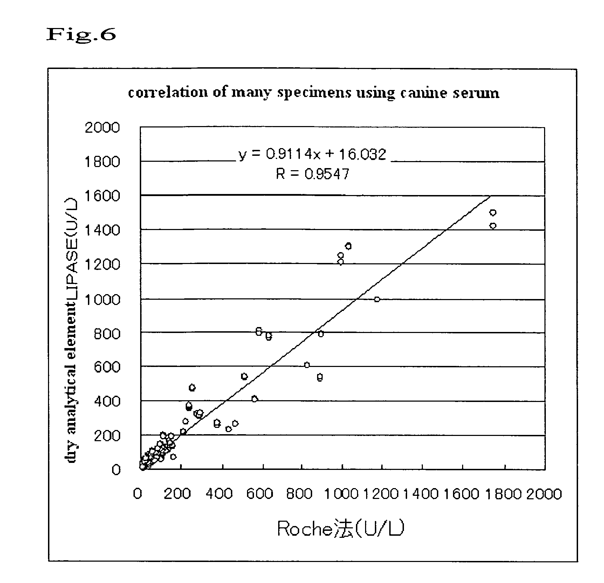

Canine serum (10µl) (N=105) was spotted onto the pancreatic lipase dry analytical element prepared in Example 1, followed by heating at 37°C. Then, lipase concentration was calculated from the changes in coloring concentration for 2 minutes (from 3 minutes to 5 minutes). The measurement apparatus used was a commercially available Fuji Dry Chem 7000. In the control method, a solution method (Roche) using 1,2-O-dilauryl-rac-glycero-3-glutaric acid/resorufin ester as a substrate was used. The results are shown in Fig.6. The result (R=0.95) was good

### Example 7: Measurement of simultaneous reproducibility

Canine serum (10µl) (N=20) was spotted onto the pancreatic lipase dry analytical element prepared in Example 1, followed by heating at 37°C. Then, lipase concentration was calculated from the changes in coloring concentration for 2 minutes (from 3 minutes to 5 minutes), and the simultaneous reproducibility (CV) and the standard deviation (SD) were determined. The measurement apparatus used was a commercially available Fuji Dry Chem 7000.

The results are shown in Table 3 below. Good results were obtained.

**Table 3:**

| Measurement Number | H level (U/L) | M level (U/L) | L level (U/L) |
|---|---|---|---|
| 1 | 1281 | 762 | 36 |
| 2 | 1317 | 742 | 34 |
| 3 | 1250 | 735 | 37 |
| 4 | 1235 | 751 | 35 |
| 5 | 1243 | 738 | 33 |
| 6 | 1252 | 721 | 35 |
| 7 | 1225 | 745 | 36 |
| 8 | 1229 | 739 | 35 |
| 9 | 1231 | 721 | 34 |
| 10 | 1274 | 728 | 36 |
| 11 | 1248 | 718 | 33 |
| 12 | 1267 | 726 | 34 |
| 13 | 1251 | 735 | 32 |
| 14 | 1269 | 735 | 34 |
| 15 | 1219 | 716 | 32 |
| 16 | 1247 | 722 | 30 |
| 17 | 1216 | 703 | 32 |
| 18 | 1228 | 714 | 33 |
| 19 | 1239 | 730 | 33 |
| 20 | 1273 | 704 | 31 |
| Number | 20 | 20 | 20 |
| Average (U/L) | 1248.5 | 730.6 | 33.9 |
| SD(U/L) | 24.8 | 15.0 | 1.8 |
| CV (%) | 2.0% | 2.1% | 5.4% |

## Claims

1. A method for producing a dry analytical element for measurement of pancreatic lipase contained in a body fluid, said element comprising at least one development layer or reagent layer containing diglyceride or triglyceride of long-chain alkyl fatty acid having 14 to 20 carbon atoms, monoglyceride lipase, and a glycerine measurement reagent, which comprises a step of adding glyceride dissolved in lower alcohol or acetone to the development layer or reagent layer and drying the glyceride.

2. The method for producing a dry analytical element according to claim 1, wherein the drying of glyceride is hot-air drying.

3. The method for producing a dry analytical element according to claim 1, wherein glyceride is dissolved in methanol, ethanol, propyl alcohol, or acetone.

4. The method for producing a dry analytical element according to claim1, wherein glyceride is triglyceride.

5. The method for producing a dry analytical element according to claim 1, wherein triglyceride is triolein.

6. The method for producing a dry analytical element according to claim 1, wherein monoglyceride lipase is *Bacillus stearothermophilus* H-165-derived monoglyceride lipase.

7. The method for producing a dry analytical element according to claim 1, wherein the glycerine measurement reagent comprises glycerol kinase, glycerophosphate oxidase, and a coloring reagent.

8. The method for producing a dry analytical element according to claim 1, wherein the dry analytical element is composed of a water-impermeable support, a reagent layer, and a development layer.

9. The method for producing a dry analytical element according to claim 1, wherein the development layer is made of fabric or a porous membrane.

10. The method for producing a dry analytical element according to claim 9, wherein the porous membrane is a porous membrane formed with polyvinylsulfone or acetylcellulose or a porous membrane formed with microbeads.

11. The method for producing a dry analytical element according to claim 9, wherein the development layer is made of fabric.

12. The method for producing a dry analytical element according to claim 1, wherein the amount of monoglyceride lipase added is 8000 U/m² to 1000 U/m².

13. A dry analytical element for measurement of pancreatic lipase contained in a body fluid, which is produced by the method according to claim 1.

14. A method for measuring pancreatic lipase in a body fluid, which comprises applying the body fluid to the dry analytical element for measurement of pancreatic lipase contained in a body fluid which is produced by the method according to claim 1.

15. The method for measuring pancreatic lipase in a body fluid according to claim 14, wherein the body fluid is canine blood.
